# EUROPEAN PATENT SPECIFICATION

(11) **EP 1 138 770 B1**
(45) Date of publication and mention of the grant of the patent: **18.10.2006**
(21) Application number: 01108166.8
(22) Date of filing: 30.03.2001
(51) Int. Cl.: C12N 15/54, C12N 9/10, C12N 15/63, C12N 5/10, C12P 7/62, C12P 21/00

(54) **Polyhydroxyalkanoate synthase and gene encoding the same enzyme**
Polyhydroxyalkanoatsynthase und dafür kodierendes Gen
Polyhydroxyalkanoatesynthase et gène codant pour celle-ci

(30) Priority: 30.03.2000 JP 2000095002
(43) Date of publication of application: 04.10.2001
(73) Proprietor: CANON KABUSHIKI KAISHA, Tokyo (JP)
(72) Inventor: Yano, Tetsuya, Ohta-ku, Tokyo (JP); Imamura, Takeshi, Ohta-ku, Tokyo (JP); Suda, Sakae, Ohta-ku, Tokyo (JP); Honma, Tsutomu, Ohta-ku, Tokyo (JP)
(74) Representative: Weser, Wolfgang

(56) References cited:
- WO-A-01/11014
- WO-A-90/12104
- US-A- 5 968 805
- MATSUSAKI HIROMI ET AL: "Cloning and molecular analysis of the poly(3-hydroxybutyrate) and poly(3-hydroxybutyrate-co-3-hydroxyalkanoa te) biosynthesis genes in Pseudomonas sp. strain 61-3" JOURNAL OF BACTERIOLOGY, WASHINGTON, DC, US, vol. 180, no. 24, December 1998 (1998-12), pages 6459-6467, XP002167124 ISSN: 0021-9193 -& DATABASE SWALL [Online] 1 May 1999 (1999-05-01) MATSUSAKI ET AL.: "PHA synthase 1" retrieved from EBI, accession no. Q9Z3Y1 Database accession no. Q9Z3Y1 XP002176214 -& DATABASE SWALL [Online] 1 May 1999 (1999-05-01) MATSUSAKI ET AL.: "PHA synthase 2" retrieved from EBI, accession no. Q9Z3X9 Database accession no. Q9Z3X9 XP002176215 -& DATABASE EM_PRO [Online] EMBL; 29 December 1998 (1998-12-29) MATSUSAKI ET AL.: "Pseudomanas sp. 61-3 genes for PHA synthase 1, PHA depolymerase, PHA synthase 2 and PhaD, complete cds." retrieved from EBI, accession no. AB014758 Datab

### SEQUENCE LISTING

<110> CANON INC.
<120> Polyhydroxyalkanoate Synthase and Gene Encoding the Same Enzyme
<130> 4051019
<160> 10
<170> Microsoft Word
<210> 1
   <211> 559
   <212> PRT
   <213> Pseudomonas cichorii YN2 ; FERM BP-7375
<400> 1
   Met Ser Asn Lys Ser Asn Asp Glu Leu Lys Tyr Gln Ala Ser Glu Asn
   1 5 10 15
   Thr Leu Gly Leu Asn Pro Val Val Gly Leu Arg Gly Lys Asp Leu Leu
   20 25 30
   Ala Ser Ala Arg Met Val Leu Arg Gln Ala Ile Lys Gln Pro Val His
   35 40 45
   Ser Val Lys His Val Ala His Phe Gly Leu Glu Leu Lys Asn Val Leu
   50 55 60
   Leu Gly Lys Ser Gly Leu Gln Pro Thr Ser Asp Asp Arg Arg Phe Ala
   65 70 75 80
   Asp Pro Ala Trp Ser Gln Asn Pro Leu Tyr Lys Arg Tyr Leu Gln Thr
   85 90 95
   Tyr Leu Ala Trp Arg Lys Glu Leu His Asp Trp Ile Asp Glu Ser Asn
   100 105 110
   Leu Ala Pro Lys Asp Val Ala Arg Gly His Phe Val Ile Asn Leu Met
   115 120 125
   Thr Glu Ala Met Ala Pro Thr Asn Thr Ala Ala Asn Pro Ala Ala Val
   130 135 140
   Lys Arg Phe Phe Glu Thr Gly Gly Lys Ser Leu Leu Asp Gly Leu Ser
   145 150 155 160
   His Leu Ala Lys Asp Leu Val His Asn Gly Gly Met Pro Ser Gln Val
   165 170 175
   Asn Met Gly Ala Phe Glu Val Gly Lys Ser Leu Gly Val Thr Glu Gly
   180 185 190
   Ala Val Val Phe Arg Asn Asp Val Leu Glu Leu Ile Gln Tyr Lys Pro
   195 200 205
   Thr Thr Glu Gln Val Tyr Glu Arg Pro Leu Leu Val Val Pro Pro Gln
   210 215 220
   Ile Asn Lys Phe Tyr Val Phe Asp Leu Ser Pro Asp Lys Ser Leu Ala
   225 230 235 240
   Arg Phe Cys Leu Arg Asn Asn Val Gln Thr Phe Ile Val Ser Trp Arg
   245 250 255
   Asn Pro Thr Lys Glu Gln Arg Glu Trp Gly Leu Ser Thr Tyr Ile Glu
   260 265 270
   Ala Leu Lys Glu Ala Val Asp Val Val Thr Ala Ile Thr Gly Ser Lys
   275 280 285
   Asp Val Asn Met Leu Gly Ala Cys Ser Gly Gly Ile Thr Cys Thr Ala
   290 295 300
   Leu Leu Gly His Tyr Ala Ala Ile Gly Glu Asn Lys Val Asn Ala Leu
   305 310 315 320
   Thr Leu Leu Val Ser Val Leu Asp Thr Thr Leu Asp Ser Asp Val Ala
   325 330 335
   Leu Phe Val Asn Glu Gln Thr Leu Glu Ala Ala Lys Arg His Ser Tyr
   340 345 350
   Gln Ala Gly Val Leu Glu Gly Arg Asp Met Ala Lys Val Phe Ala Trp
   355 360 365
   Met Arg Pro Asn Asp Leu Ile Trp Asn Tyr Trp Val Asn Asn Tyr Leu
   370 375 380
   Leu Gly Asn Glu Pro Pro Val Phe Asp Ile Leu Phe Trp Asn Asn Asp
   385 390 395 400
   Thr Thr Arg Leu Pro Ala Ala Phe His Gly Asp Leu Ile Glu Leu Phe
   405 410 415
   Lys Asn Asn Pro Leu Ile Arg Pro Asn Ala Leu Glu Val Cys Gly Thr
   420 425 430
   Pro Ile Asp Leu Lys Gln Val Thr Ala Asp Ile Phe Ser Leu Ala Gly
   435 440 445
   Thr Asn Asp His Ile Thr Pro Trp Lys Ser Cys Tyr Lys Ser Ala Gln
   450 455 460
   Leu Phe Gly Gly Asn Val Glu Phe Val Leu Ser Ser Ser Gly His Ile
   465 470 475 480
   Gln Ser Ile Leu Asn Pro Pro Gly Asn Pro Lys Ser Arg Tyr Met Thr
   485 490 495
   Ser Thr Glu Val Ala Glu Asn Ala Asp Glu Trp Gln Ala Asn Ala Thr
   500 505 510
   Lys His Thr Asp Ser Trp Trp Leu His Trp Gln Ala Trp Gln Ala Gln
   515 520 525
   Arg Ser Gly Glu Leu Lys Lys Ser Pro Thr Lys Leu Gly Ser Lys Ala
   530 535 540
   Tyr Pro Ala Gly Glu Ala Ala Pro Gly Thr Tyr Val His Glu Arg
   545 550 555
<210> 2
   <211> 1680
   <212> DNA
   <213> Pseudomonas cichorii YN2 ; FERM BP-7375
<400> 2
   atgagtaaca agagtaacga tgagttgaag tatcaagcct ctgaaaacac 50
   cttggggctt aatcctgtcg ttgggctgcg tggaaaggat ctactggctt 100
   ctgctcgaat ggtgcttagg caggccatca agcaaccggt gcacagcgtc 150
   aaacatgtcg cgcactttgg tcttgaactc aagaacgtac tgctgggtaa 200
   atccgggctg caaccgacca gcgatgaccg tcgcttcgcc gatccggcct 250
   ggagccagaa cccgctctat aaacgttatt tgcaaaccta cctggcgtgg 300
   cgcaaggaac tccacgactg gatcgatgaa agtaacctcg cccccaagga 350
   tgtggcgcgt gggcacttcg tgatcaacct catgaccgaa gccatggcgc 400
   cgaccaacac cgcggccaac ccggcggcag tcaaacgctt tttcgaaacc 450
   ggtggcaaaa gcctgctcga cggcctctcg cacctggcca aggatctggt 500
   acacaacggc ggcatgccga gccaggtcaa catgggtgca ttcgaggtcg 550
   gcaagagcct gggcgtgacc gaaggcgcgg tggtgtttcg caacgatgtg 600
   ctggaactga tccagtacaa gccgaccacc gagcaggtat acgaacgccc 650
   gctgctggtg gtgccgccgc agatcaacaa gttctacgtt ttcgacctga 700
   gcccggacaa gagcctggcg cggttctgcc tgcgcaacaa cgtgcaaacg 750
   ttcatcgtca gctggcgaaa tcccaccaag gaacagcgag agtggggcct 800
   gtcgacctac atcgaagccc tcaaggaagc ggttgatgtc gttaccgcga 850
   tcaccggcag caaagacgtg aacatgctcg gcgcctgctc cggcggcatc 900
   acttgcaccg cgctgctggg ccattacgcg gcgattggcg aaaacaaggt 950
   caacgccctg accttgctgg tgagcgtgct tgataccacc ctcgacagcg 1000
   atgttgccct gttcgtcaat gaacagaccc ttgaagccgc caagcgccac 1050
   tcgtaccagg ccggcgtact ggaaggccgc gacatggcga aggtcttcgc 1100
   ctggatgcgc cccaacgatc tgatctggaa ctactgggtc aacaattacc 1150
   tgctaggcaa cgaaccgccg gtgttcgaca tcctgttctg gaacaacgac 1200
   accacacggt tgcccgcggc gttccacggc gacctgatcg aactgttcaa 1250
   aaataaccca ctgattcgcc cgaatgcact ggaagtgtgc ggcaccccca 1300
   tcgacctcaa gcaggtgacg gccgacatct tttccctggc cggcaccaac 1350
   gaccacatca ccccgtggaa gtcctgctac aagtcggcgc aactgtttgg 1400
   cggcaacgtt gaattcgtgc tgtcgagcag cgggcatatc cagagcatcc 1450
   tgaacccgcc gggcaatccg aaatcgcgct acatgaccag caccgaagtg 1500
   gcggaaaatg ccgatgaatg gcaagcgaat gccaccaagc ataccgattc 1550
   ctggtggctg cactggcagg cctggcaggc ccaacgctcg ggcgagctga 1600
   aaaagtcccc gacaaaactg ggcagcaagg cgtatccggc aggtgaagcg 1650
   gcgccaggca cgtacgtgca cgaacggtaa 1680
<210> 3
   <211> 560
   <212> PRT
   <213> Pseudomonas cichorii YN2 ; PERM BP-7375
<400> 3
   Met Arg Asp Lys Pro Ala Arg Glu Ser Leu Pro Thr Pro Ala Lys Phe
   1 5 10 15
   Ile Asn Ala Gln Ser Ala Ile Thr Gly Leu Arg Gly Arg Asp Leu Val
   20 25 30
   Ser Thr Leu Arg Ser Val Ala Ala His Gly Leu Arg His Pro Val His
   35 40 45
   Thr Ala Arg His Ala Leu Lys Leu Gly Gly Gln Leu Gly Arg Val Leu
   50 55 60
   Leu Gly Asp Thr Leu His Pro Thr Asn Pro Gln Asp Arg Arg Phe Asp
   65 70 75 80
   Asp Pro Ala Trp Ser Leu Asn Pro Phe Tyr Arg Arg Ser Leu Gln Ala
   85 90 95
   Tyr Leu Ser Trp Gln Lys Gln Val Lys Ser Trp Ile Asp Glu Ser Asn
   100 105 110
   Met Ser Pro Asp Asp Arg Ala Arg Ala His Phe Ala Phe Ala Leu Leu
   115 120 125
   Asn Asp Ala Val Ser Pro Ser Asn Ser Leu Leu Asn Pro Leu Ala Ile
   130 135 140
   Lys Glu Ile Phe Asn Ser Gly Gly Asn Ser Leu Val Arg Gly Ile Gly
   145 150 155 160
   His Leu Val Asp Asp Leu Leu His Asn Asp Gly Leu Pro Arg Gln Val
   165 170 175
   Thr Arg His Ala Phe Glu Val Gly Lys Thr Val Ala Thr Thr Thr Gly
   180 185 190
   Ala Val Val Phe Arg Asn Glu Leu Leu Glu Leu Ile Gln Tyr Lys Pro
   195 200 205
   Met Ser Glu Lys Gln Tyr Ser Lys Pro Leu Leu Val Val Pro Pro Gln
   210 215 220
   Ile Asn Lys Tyr Tyr Ile Phe Asp Leu Ser Pro His Asn Ser Phe Val
   225 230 235 240
   Gln Phe Ala Leu Lys Asn Gly Leu Gln Thr Phe Val Ile Ser Trp Arg
   245 250 255
   Asn Pro Asp Val Arg His Arg Glu Trp Gly Leu Ser Thr Tyr Val Glu
   260 265 270
   Ala Val Glu Glu Ala Met Asn Val Cys Arg Ala Ile Thr Gly Ala Arg
   275 280 285
   Glu Val Asn Leu Met Gly Ala Cys Ala Gly Gly Leu Thr Ile Ala Ala
   290 295 300
   Leu Gln Gly His Leu Gln Ala Lys Arg Gln Leu Arg Arg Val Ser Ser
   305 310 315 320
   Ala Thr Tyr Leu Val Ser Leu Leu Asp Ser Gln Leu Asp Ser Pro Ala
   325 330 335
   Thr Leu Phe Ala Asp Glu Gln Thr Leu Glu Ala Ala Lys Arg Arg Ser
   340 345 350
   Tyr Gln Lys Gly Val Leu Glu Gly Arg Asp Met Ala Lys Val Phe Ala
   355 360 365
   Trp Met Arg Pro Asn Asp Leu Ile Trp Ser Tyr Phe Val Asn Asn Tyr
   370 375 380
   Leu Met Gly Lys Glu Pro Pro Ala Phe Asp Ile Leu Tyr Trp Asn Asn
   385 390 395 400
   Asp Asn Thr Arg Leu Pro Ala Ala Leu His Gly Asp Leu Leu Asp Phe
   405 410 415
   Phe Lys His Asn Pro Leu Ser His Pro Gly Gly Leu Glu Val Cys Gly
   420 425 430
   Thr Pro Ile Asp Leu Gln Lys Val Thr Val Asp Ser Phe Ser Val Ala
   435 440 445
   Gly Ile Asn Asp His Ile Thr Pro Trp Asp Ala Val Tyr Arg Ser Thr
   450 455 460
   Leu Leu Leu Gly Gly Glu Arg Arg Phe Val Leu Ala Asn Ser Gly His
   465 470 475 480
   Val Gln Ser Ile Leu Asn Pro Pro Asn Asn Pro Lys Ala Asn Tyr Leu
   485 490 495
   Glu Gly Ala Lys Leu Ser Ser Asp Pro Arg Ala Trp Tyr Tyr Asp Ala
   500 505 510
   Lys Pro Val Asp Gly Ser Trp Trp Thr Gln Trp Leu Gly Trp Ile Gln
   515 520 525
   Glu Arg Ser Gly Ala Gln Lys Glu Thr His Met Ala Leu Gly Asn Gln
   530 535 540
   Asn Tyr Pro Pro Met Glu Ala Ala Pro Gly Thr Tyr Val Arg Val Arg
   545 550 555 560
<210> 4
   <211> 1683
   <212> DNA
   <213> Pseudomonas cichorii YN2 ; FERM BP-7375
<400> 4
   atgcgcgata aacctgcgag ggagtcacta cccacccccg ccaagttcat 50
   caacgcacaa agtgcgatta ccggcctgcg tggccgggat ctggtttcga 100
   ctttgcgcag tgtcgccgcc catggcctgc gccaccccgt gcacaccgcg 150
   cgacacgcct tgaaactggg tggtcaactg ggacgcgtgt tgctgggcga 200
   caccctgcat cccaccaacc cgcaagaccg tcgcttcgac gatccggcgt 250
   ggagtctcaa tcccttttat cgtcgcagcc tgcaggcgta cctgagctgg 300
   cagaagcagg tcaagagctg gatcgacgaa agcaacatga gcccggatga 350
   ccgcgcccgt gcgcacttcg cgttcgccct gctcaacgat gccgtgtcgc 400
   cgtccaacag cctgctcaat ccgctggcga tcaaggaaat cttcaactcc 450
   ggcggcaaca gcctggtgcg cgggatcggc catctggtcg atgacctctt 500
   gcacaacgat ggcttgcccc ggcaagtcac caggcatgca ttcgaggttg 550
   gcaagaccgt cgccaccacc accggcgccg tggtgtttcg caacgagctg 600
   ctggagctga tccaatacaa gccgatgagc gaaaagcagt attccaaacc 650
   gctgctggtg gtgccgccac agatcaacaa gtactacatt tttgacctca 700
   gcccccataa cagcttcgtc cagttcgcgc tcaagaacgg cctgcaaacc 750
   ttcgtcatca gctggcgcaa tccggatgta cgtcaccgcg aatggggcct 800
   gtcgacctac gtcgaagcgg tggaagaagc catgaatgtc tgccgggcaa 850
   tcaccggcgc gcgcgaggtc aacctgatgg gcgcctgcgc tggcgggctg 900
   accattgctg ccctgcaggg ccacttgcaa gccaagcgac agctgcgccg 950
   cgtctccagc gcgacgtacc tggtgagcct gctcgacagc caactggaca 1000
   gcccggccac actcttcgcc gacgaacaga ccctggaggc ggccaagcgc 1050
   cgctcctacc agaaaggtgt gctggaaggc cgcgacatgg ccaaggtttt 1100
   cgcctggatg cgccccaacg atttgatctg gagctacttc gtcaacaatt 1150
   acctgatggg caaggagccg ccggcgttcg acattctcta ctggaacaat 1200
   gacaacacac gcctgccggc cgccctgcat ggtgacttgc tggacttctt 1250
   caagcacaac ccgctgagcc atccgggtgg cctggaagtg tgcggcaccc 1300
   cgatcgactt gcaaaaggtc accgtcgaca gtttcagcgt ggccggcatc 1350
   aacgatcaca tcacgccgtg ggacgcggtg tatcgctcaa ccctgttgct 1400
   cggtggcgag cgtcgctttg tcctggccaa cagcggtcat gtgcagagca 1450
   ttctcaaccc gccgaacaat ccgaaagcca actacctcga aggtgcaaaa 1500
   ctaagcagcg accccagggc ctggtactac gacgccaagc ccgtcgacgg 1550
   tagctggtgg acgcaatggc tgggctggat tcaggagcgc tcgggcgcgc 1600
   aaaaagaaac ccacatggcc ctcggcaatc agaattatcc accgatggag 1650
   gcggcgcccg ggacttacgt gcgcgtgcgc tga 1683
<210> 5
   <211>20
   <212> DNA
   <213> Artificial Sequence
<220>
   <223> Primer for PCR multiplication
<400> 5
   tgctggaact gatccagtac 20
<210> 6
   <211> 23
   <212> DNA
   <213> Artificial Sequence
<220>
   <223> Primer for PCR multiplication
<400> 6
   gggttgagga tgctctggat gtg 23
<210> 7
   <211> 29
   <212> DNA
   <213> Artificial Sequence
   <220>
   <223> Primer for PCR multiplication
<400> 7
   ggaccaagct tctcgtctca gggcaatgg 29
<210> 8
   <211> 29
   <212> DNA
   <213> Artificial Sequence
<220>
   <223> Primer for PCR multiplication
<400> 8
   cgagcaagct tgctcctaca ggtgaaggc 29
<210> 9
   <211> 29
   <212> DNA
   <213> Artificial Sequence
<220>
   <223> Primer for PCR multiplication
<400> 9
   gtattaagct tgaagacgaa ggagtgttg 29
<210> 10
   <211> 30
   <212> DNA
   <213> Artificial Sequence
<220>
   <223> Primer for PCR multiplication
<400> 10
   catccaagct tcttatgatc gggtcatgcc 30

## Description

### BACKGROUND OF THE INVENTION

### Field of the Invention

This invention relates to a polyhydroxyalkanoate (hereinafter, referred to as a "PHA") synthase, a gene encoding the PHA synthase, a recombinant vector containing the gene, a transformant capable of expressing the PHA synthase which has been transformed by the recombinant vector, a process for producing the PHA synthase utilizing the transformant, and a process for preparing the PHA utilizing the transformant. In particular, this invention relates to a microorganism-derived PHA synthase capable of producing a polyhydroxyalkanoate and a gene encoding the PHA synthase utilized for expressing the PHA synthase by transformation.

### Related Background Art

There have been reported a number of microorganisms producing poly-3-hydroxybutyric acid (PHB) or another PHA and storing it therein ("Biodegradable Plastic Handbook", edited by Biodegradative Plastic Research Society, NTS Co. Ltd., p.178-197). These polymers may be, as conventional plastics, used for producing a variety of products by, for example, melt-processing. Since they are biodegradable, they have an advantage that they can be completely degraded by microorganisms in the natural environment, and they do not cause pollution due to remaining in the natural environment like many conventional polymer compounds. Furthermore, they are excellently biocompatible, and thus are expected to be used in applications such as a medical soft member.

It is known that a composition and a structure of such a PHA produced by a microorganism may considerably vary depending on the type of a microorganism used for the production, a culture-medium composition and culturing conditions. Investigations have been, therefore, mainly focused on controlling such a composition or structure for the purpose of improving physical properties of a PHA.

For example, Japanese Patent Application Nos. 6-15604, 7-14352 and 8-19227 describe that Alcaligenes eutropus H16 (ATCC No. 17699) and its variants may produce 3-hydroxybutyric acid (3HB) and its copolymer with 3-hydroxyvaleric acid (3HV) with various composition ratios by changing a carbon source during culturing.

Japanese Patent Publication No. 2642937 discloses that PHA in which a monomer unit is 3-hydroxyalkanoate with 6 to 12 carbon atoms may be produced by supplying a non-cyclic aliphatic hydrocarbon as a carbon source to Pseudomonas oleovorans (ATCC No. 29347).

Japanese Patent Application Laid-Open No. 5-74492 discloses methods in which Methylobaterium sp., Paracoccus sp., Alcaligenes sp., and Pseudomonas sp. are contacted with a primary alcohol with 3 to 7 carbon atoms to produce a copolymer of 3HB and 3HV.

Japanese Patent Application Laid-Open Nos. 5-93049 and 7-265065 disclose that Aeromonas caviae is cultured using oleic acid or olive oil as a carbon source to produce a two-component copolymer of 3HB and 3-hydroxyhexanoic acid (3HHx).

Japanese Patent Application Laid-Open No. 9-191893 discloses that Comamonas acidovorans IF013852 is cultured using gluconic acid and 1,4-butanediol as carbon sources to produce a polyester having 3HB and 4-hydroxybutyric acid as monomer units.

J. of Bacteriology 180(1998), pp. 6459-6467, discloses that Pseudomonas sp. Strain 61-3, which produces a blend of [P(3HB)] homopolymer and a random copolymer [P(3HB-co-3HA] consisting of 3HA units of 4 to 12 carbon atoms, were cloned and analyzed at the molecular level.

Furthermore, it is reported that certain microorganisms produce PHAs having a variety of substituents such as unsaturated hydrocarbon, ester, aryl (aromatic), and cyans groups, halogenated hydrocarbon and epoxide. Recently, there have been attempts for improving physical properties of a PHA produced by a microorganism using such a procedure. For example, Makromol. Chem., 191, 1957 - 1965 (1990); Macromolecules, 24, 5256-5260 (1991); and Chirality, 3, 492-494 (1991) describe production of a PHA comprising 3-hydroxy-5-phenylvaleric acid (3HPV) as a monomer unit by Pseudomonas oleovorans, where variations in polymer physical properties probably due to the presence of 3HPV were observed.

As described above, microorganism-produced PHAs with various combinations of composition and structure have been obtained by varying factors such as the type of a microorganism used, a culture medium composition and culturing conditions. Each microorganism has an intrinsic PHA synthase with a substrate specificity which is significantly different from others. Thus, it has been difficult to produce PHAs comprising different monomer units suitable to a variety of applications using known microorganisms or PHA synthases in such known microorganisms.

Meanwhile, as described above, a PHA having a variety of substituents in its side chains may be expected to be a "functional polymer" having significantly useful functions and properties owing to the properties of the introduced substituents. It is, therefore, extremely useful and important to search and develop a microorganism which can produce and store a very useful polymer having both such functionality and biodegradability. Furthermore, identification of a PHA synthase involved in production of the highly useful PHA and obtaining a gene encoding the PHA synthase may allow us to produce a novel transformed microorganism capable of producing a desired PHA. That is, constructing a recombinant vector comprising a gene encoding a PHA synthase and providing a microorganism transformed by the recombinant vector may allow us to prepare a PHA using the transformed microorganism or to express a recombinant type of PHA synthase. As described above, it may be important that a transformed microorganism is used to prepare a desired PHA for providing a highly useful tool for improving a productivity for the PHA and for promoting utilization of the PHA.

### SUMMARY OF THE INVENTION

Objects of this invention which can solve the above problems are to search a novel microorganism capable of producing and storing in microorganisms a PHA having a novel side-chain structure, to identify an enzyme protein related to the ability of producing the novel PHA, i.e., a novel PHA synthase, and to determine a gene encoding its amino acid sequence. More specifically, an object of the present invention is to provide a novel PHA synthase derived from a microorganism producing a PHA having a novel side chain structure and a DNA encoding its amino acid sequence. Another object of this invention is to provide a recombinant vector to which a DNA encoding an available PHA synthase is introduced and which is used for transformation of a microorganism and a transformed microorganism produced using the recombinant vector. A further object of this invention is to provide a process for expressing and producing a recombinant PHA synthase in the transformed microorganism and a process for preparing a desired PHA using the transformed microorganism.

Still another object of this invention is to provide a modified PHA synthase in which its amino acid sequence is modified as long as an enzyme activity is not affected in expression of the recombinant PHA synthase in the transformed microorganism as described above and a DNA encoding the modified amino acid sequence.

For developing a PHA having a novel side-chain structure useful as, for example, a device material or a medical material aiming at solving the above problems, the inventors have searched a novel microorganism capable of producing and storing the desired PHA therein. Additionally, the inventors have intensely investigated selected novel microorganisms producing a novel PHA for identifying a PHA synthase involved in production of the novel PHA and for obtaining a gene encoding the PHA synthase. Furthermore, the inventors have conducted investigation for constructing a recombinant vector with a gene for the obtained PHA synthase, transforming a host microorganism using the recombinant vector, expressing a recombinant PHA synthase in the transformed microorganism obtained and determining production of the desired PHA.

In the course of the above investigation, the inventors synthesized 5-(4-fluorophenyl) valeric acid (FPVA) represented by formula (II): 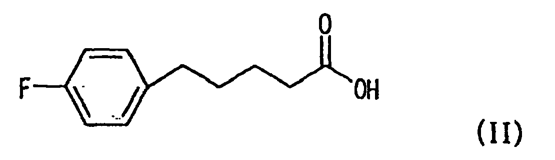 and separated from a soil a novel microorganism capable of converting the above compound (II) as a starting material (substrate) into corresponding 3-hydroxy-5-(4-fluorophenyl)valeric acid (3HFPV) represented by formula (III): 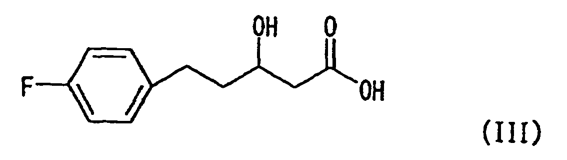 and producing and storing a novel PHA with a monomer unit represented by formula (I): 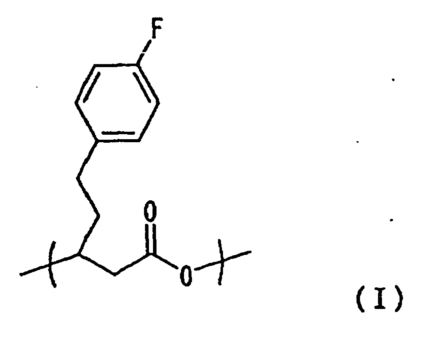 derived from 3HFPV. The novel microorganism separated is designated as YN2 strain. The inventors have also found that in addition to the above enzymatic activity for converting FPVA into 3HFPV, the YN2 strain may also use 4-cyclohexylbutyric acid (CHxBA) represented by formula (IV): 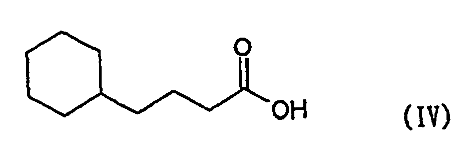 as a starting material (substrate) to convert it into 3-hydroxy-4-cyclohexylbutyric acid (3HCHxB) represented by formula (V): 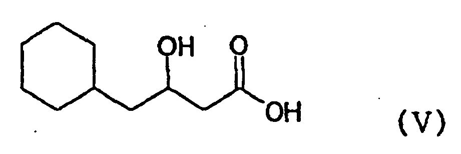 and to produce and store a PHA with a monomer unit represented by formula (VI): 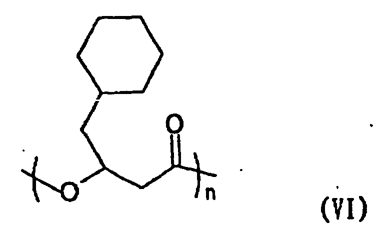 derived from 3HCHxB.

Microbiological properties of YN2 strain are as follows.

### <Microbiological properties of YN2 strain>

### (Morphologic properties)

Cell shape and size: Bacilliform, 0.8 µm × 1.0 to 1.2 µm
Cell polymorphism: No
Motility: Yes
Sporulation: No
Gram stainability: Negative
Colonization: Circular, smooth in the overall periphery, low convex, smooth surface, gloss, yellowish white

### (Physiological properties)

Catalase: Positive
Oxidase: Positive
O/F test: oxidized form
Reduction of a nitrate: Negative
Indole formation: Negative
Acidification of dextrose: Negative
Arginine dihydrolase: Negative
Urease: Negative
Esculin hydrolysis: Negative
Gelatin hydrolysis: Negative
β-Galactosidase: Negative
Fluorochrome production on King's B agar: Positive Formation with 4% NaCl: Negative
Accumulation of poly-β-hydroxybuatyric acid: Negative

### (Substrate assimilation ability)

Dextrose: Positive
L-Arabinose: Negaative
D-Mannose: Positive
D-Mannitol: Positive
N-Acetyl-D-glucosamine: Positive
Maltose: Negative
Potassium gluconate: Positive
n-Capric acid: Positive
Adipic acid: Negative
dl-Malic acid: Positive
Sodium citrate: Positive
Phenyl acetate: Positive

From these microbiological properties, the inventors have attempted to categorize YN2 strain according to Bergey's Manual of Systematic Bacteriology, Volume 1 (1984) and Bergey's Manual of Determinative Bacteriology 9th ed. (1994) to determine that the strain belongs to Pseudomonas cichorii. Thus, it was designated as Pseudomonas cichorii YN2.

There have been no reports on a strain in Pseudomonas cichorii capable of producing a PHA as exhibited by YN2 strain. The inventors have, therefore, determined that YN2 strain is a novel microorganism. The applicant deposited Pseudomonas cichorii YN2 to Patent Microorganism Depository Center in the National Institute of Bioscience and Human Technology, Agency of Industrial Science and Technology, Ministry of International Trade and Industry, under the deposition number of FERM BP-7375. YN2 strain has been internationally deposited on the basis of the Budapest Treaty, and its international accession number is "FERM BP-7375".

The inventors achieved cloning a gene for a PHA synthase from the novel microorganism YN2 strain and sequenced the gene. The inventors also determined an amino acid sequence for the PHA synthase encoded by the gene. Based on the above observation, the present invention was achieved.

Specifically, a PHA synthase of the present invention is a polyhydroxyalkanoate synthase having an amino acid sequence of SEQ ID NO. 1 or 3. Furthermore, the PHA synthase of the present invention may be a PHA synthase substantially retaining the amino acid sequence of SEQ ID NO. 1 and having a modified amino acid sequence where amino acids are deleted, substituted or added as long as it does not deteriorate an activity as the polyhydroxyalkanoate synthase, or a PHA synthase substantially retaining the amino acid sequence of SEQ ID NO. 3 and having a modified amino acid sequence where amino acids are deleted, substituted or added as long as it does not deteriorate activity as the polyhydroxyalkanoate synthase.

A PHA synthase gene of the present invention is a gene for a polyhydroxyalkanoate synthase comprising a DNA encoding the amino acid sequence of SEQ ID NO. 1 or the sequence of its modified amino acid, or a gene for a polyhydroxyalkanoate synthase comprising a DNA encoding the amino acid sequence of SEQ ID NO. 3 or the sequence of its modified amino acid. Embodiments of a PHA synthase gene of the present invention derived from a genome gene in YN2 strain include a PHA synthase gene comprising a DNA sequence of SEQ ID NO. 2 as a DNA encoding the amino acid sequence of SEQ ID NO. 1 and a PHA synthase gene comprising a DNA sequence of SEQ ID NO. 4 as a DNA encoding the amino acid sequence of SEQ ID NO. 3.

This invention also provides a recombinant vector comprising a gene DNA encoding the above amino acid sequence as a polyhydroxyalkanoate synthase gene. This invention also provides a transformed microorganism transformed by introducing a recombinant vector adapted to a host.

The present invention also provides a process for preparing a polyhydroxyalkanoate comprising the steps of culturing the transformed microorganism to which a recombinant vector has been introduced in a culture medium containing a substrate for a polyhydroxyalkanoate synthase and collecting the polyhydroxyalkanoate from the culture preparation. The present invention also provides a process for producing a polyhydroxyalkanoate comprising the steps of culturing the transformed microorganism to which a recombinant vector has been introduced and making the transformed microorganism produce the polyhydroxyalkanoate.

A preferable process for producing a polyhydroxyalkanoate may utilize substrate specificity characteristic of a polyhydroxyalkanoate synthase derived from YN2 strain: for example, preparation of a polyhydroxyalkanoate comprising a monomer unit represented by formula (I) derived from 3HFPV utilizing the above transformed microorganism or preparation of a polyhydroxyalkanoate comprising a monomer unit represented by formula (VI) derived from 3HCHxB.

A PHA synthase and a gene encoding the PHA synthase of the present invention are derived from a novel microorganism, Pseudomonas cichorii YN2 strain and exhibits such substrate specificity that it selectively produces a PHA comprising a monomer unit having a novel side chain structure. A recombinant vector comprising the PHA synthase gene and a microorganism transformed by the recombinant vector are capable of producing a PHA exhibiting substrate specificity similar to Pseudomonas cichorii YN2. Thus, a PHA synthase gene of this invention encodes an enzyme which permits preparation of a PHA selectively comprising a monomer unit having a novel side-chain structure and allows us to create a transformed microorganism useful for preparing a PHA having various useful physical properties which may be expected to be applied to a functional polymer.

### BRIEF DESCRIPTION OF THE DRAWINGS

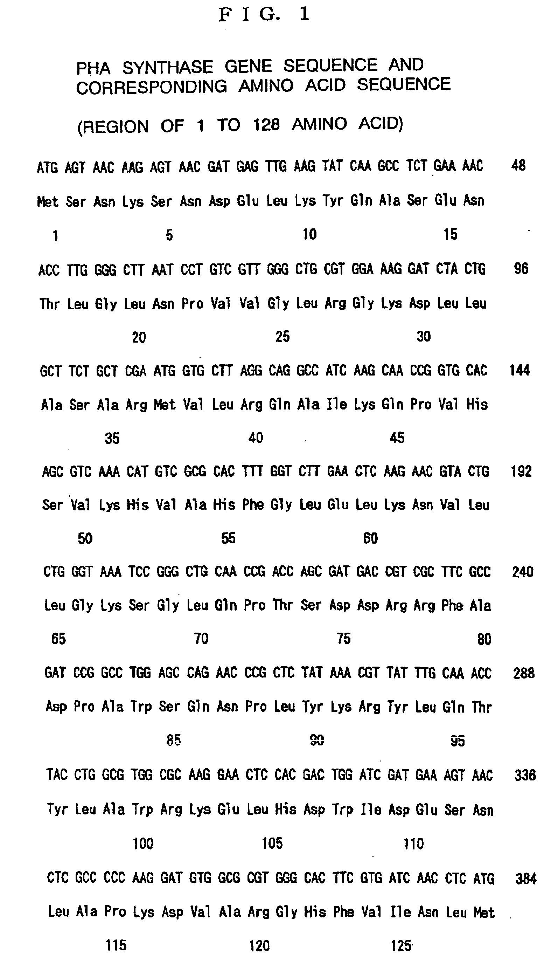

FIG. 1 illustrates a base sequence encoding the first PHA synthase derived from Pseudomonas cichorii YN2 (FERM BP-7375);

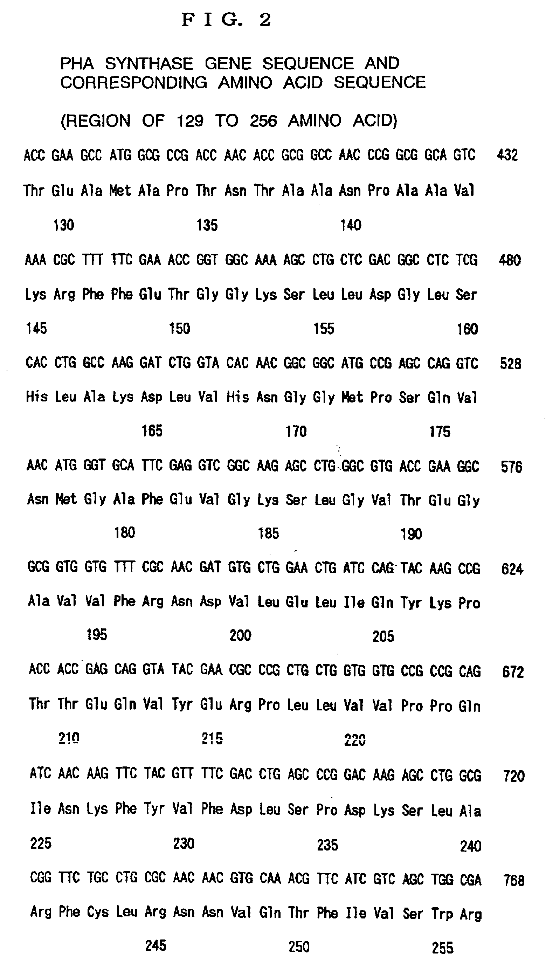

FIG. 2 illustrates a base sequence encoding the first PHA synthase derived from Pseudomonas cichorii YN2 (FERM BP-7375);

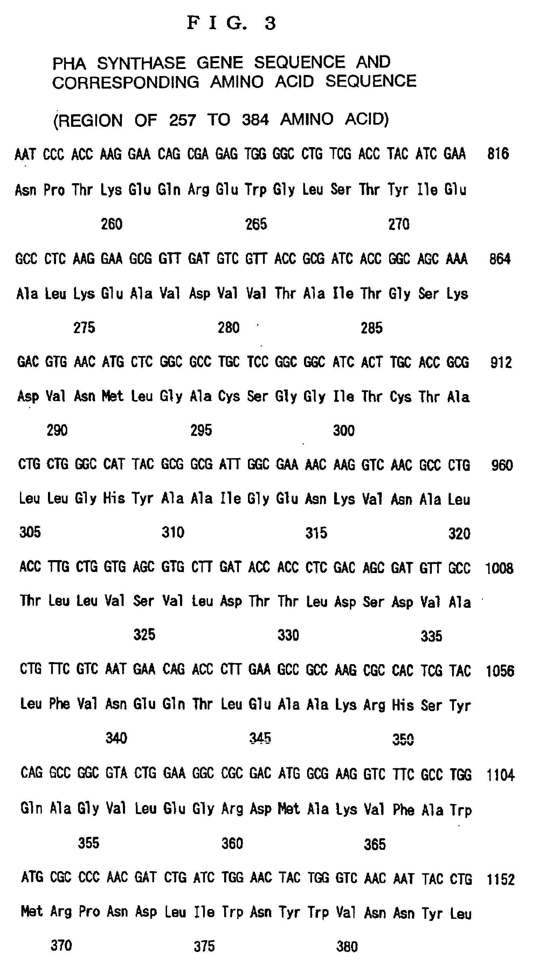

FIG. 3 illustrates a base sequence encoding the first PHA synthase derived from Pseudomonas cichorii YN2 (FERM BP-7375);

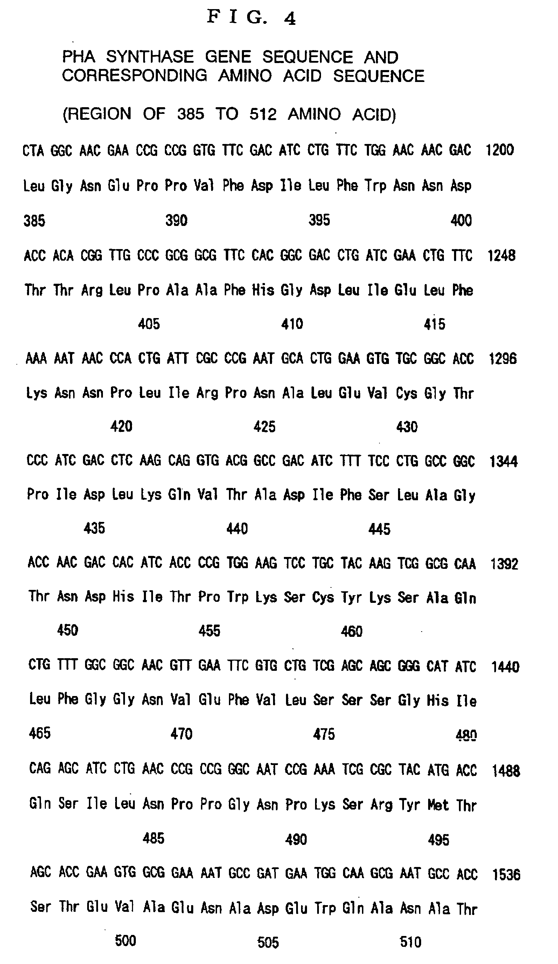

FIG. 4 illustrates a base sequence encoding the first PHA synthase derived from Pseudomonas cichorii YN2 (FERM BP-7375);

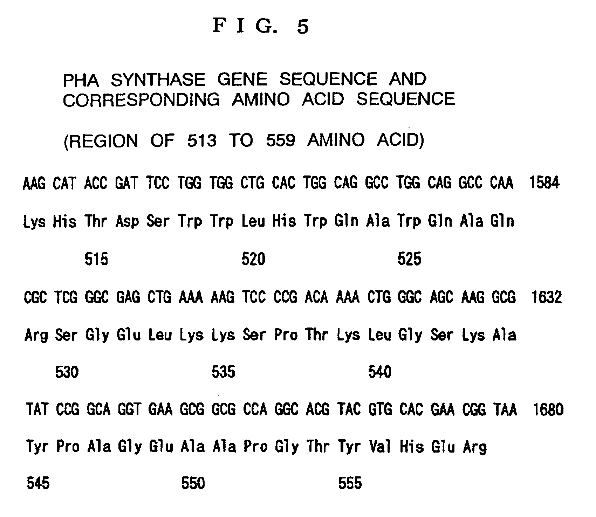

FIG. 5 illustrates a base sequence encoding the first PHA synthase derived from Pseudomonas cichorii YN2 (FERM BP-7375);

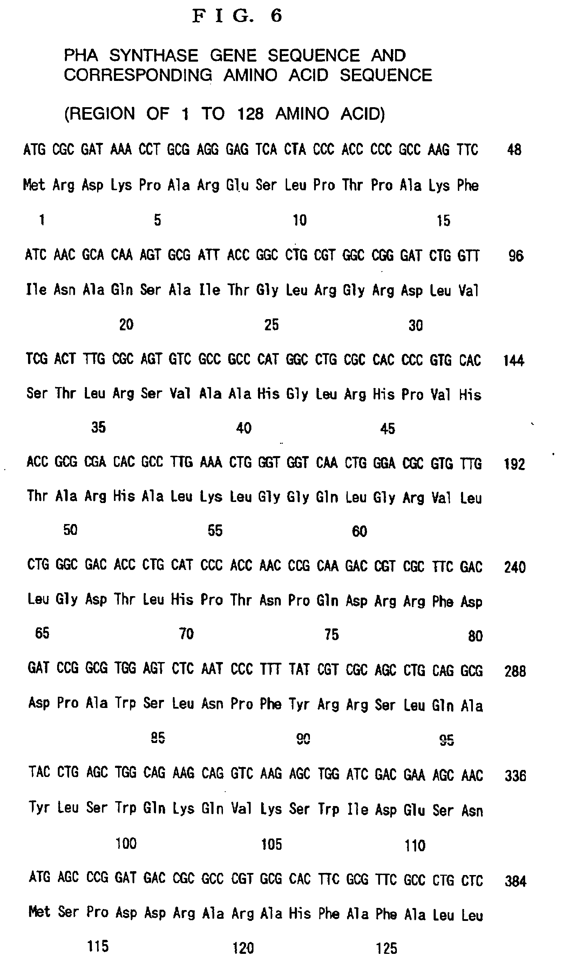

FIG. 6 illustrates a base sequence encoding the second PHA synthase derived from Pseudomonas cichorii YN2 (FERM BP-7375);

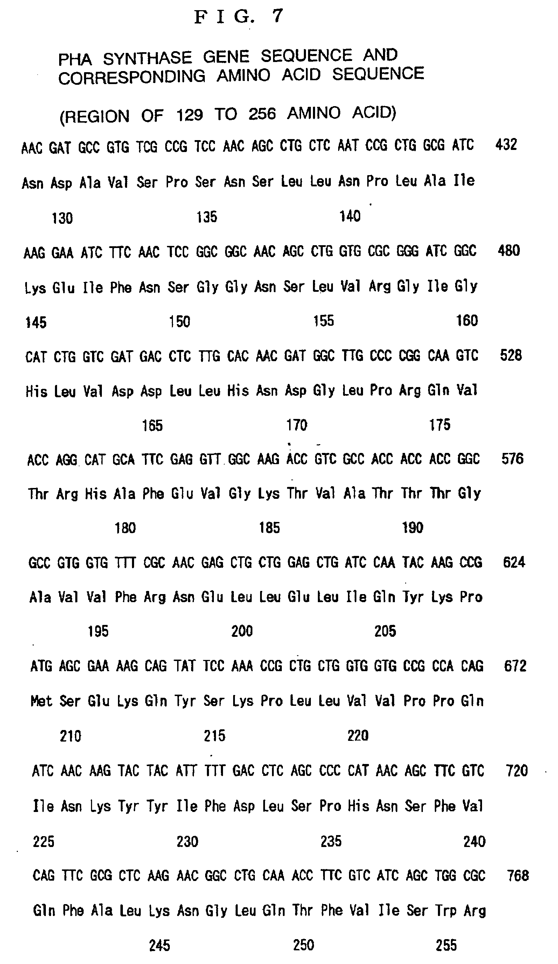

FIG. 7 illustrates a base sequence encoding the second PHA synthase derived from Pseudomonas cichorii YN2 (FERM BP-7375);

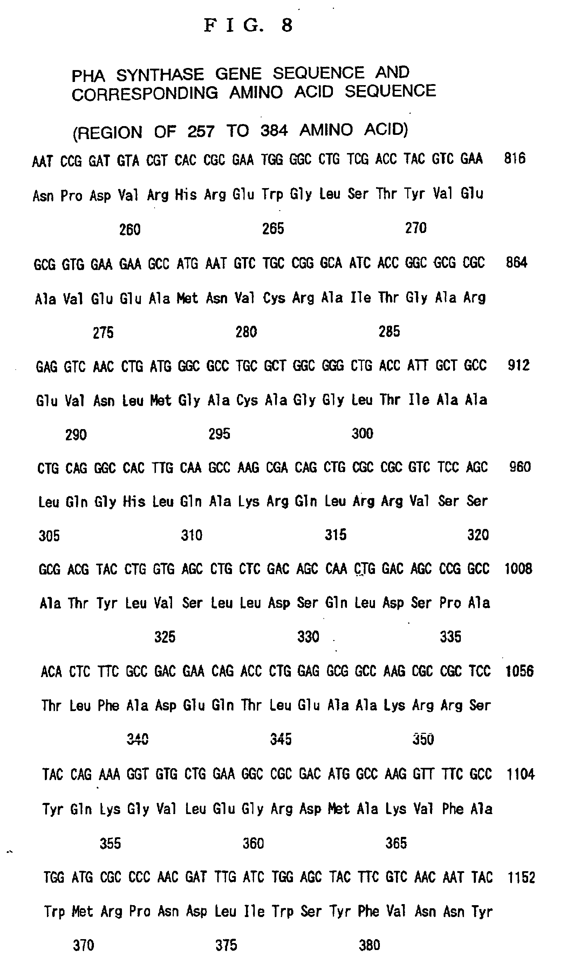

FIG. 8 illustrates a base sequence encoding the second PHA synthase derived from Pseudomonas cichorii YN2 (FERM BP-7375);

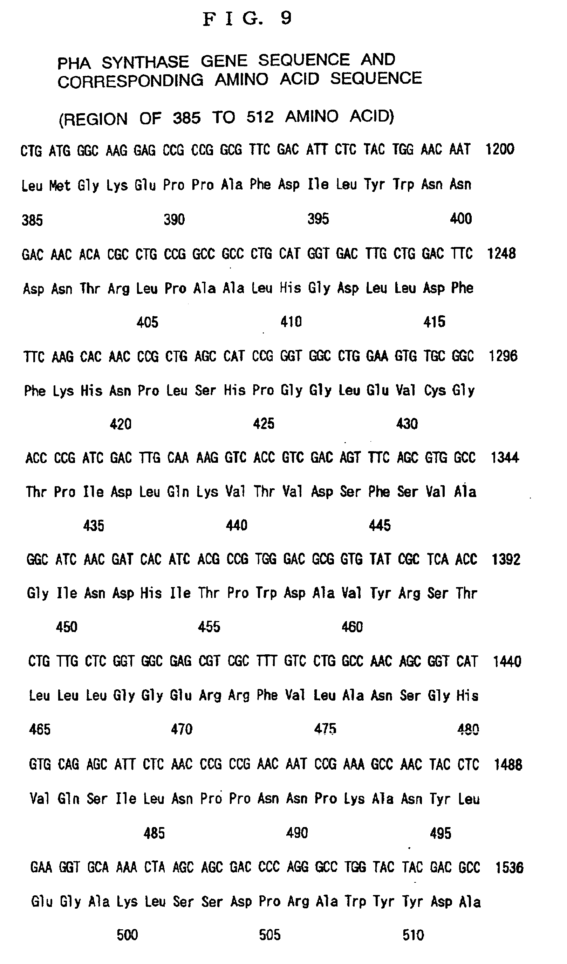

FIG. 9 illustrates a base sequence encoding the second PHA synthase derived from Pseudomonas cichorii YN2 (FERM BP-7375); and

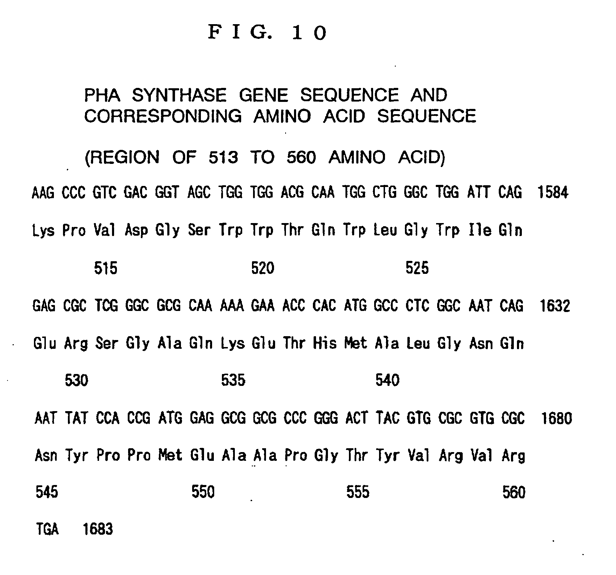

FIG. 10 illustrates a base sequence encoding the second PHA synthase derived from Pseudomonas cichorii YN2 (FERM BP-7375).

### DETAILED DESCRIPTION OF THE PREFERRED EMBODIMENTS

A PHA synthase of this invention is an enzyme protein derived from a novel microorganism isolated by the present inventors, Pseudomonas cichorii YN2 (FERM BP-7375). Specifically, it can covert 5-(4-fluorophenyl)valeric acid (FPVA) into corresponding 3-hydroxy-5-(4-fluorophenyl)valeric acid (3HFPV) or 4-cyclohexylbutyric acid (CHxBA) into corresponding 3-hydroxy-4-cyclohexylbutyric acid (3HCHxB) and thus has enzymatic activity involved in production of a PHA comprising a corresponding monomer unit.

A PHA synthase and a gene encoding the enzyme of this invention will be more specifically described.

From YN2 strain, the inventors have cloned a gene translated into a PHA synthase which exhibits the above substrate specificity, to determine the presence of a PHA synthase comprising at least two amino acid sequences. Specifically, a PHA synthase of this invention in a chromogene in YN2 strain comprises two enzymes, i.e., a PHA synthase comprising the amino acid sequence of SEQ ID NO. 1 encoded by a DNA having the sequence of SEQ ID NO. 2 and a PHA synthase comprising the amino acid sequence of SEQ ID NO. 3 encoded by a DNA having the sequence of SEQ ID NO. 4. Gene DNAs of the sequences of SEQ ID NOs. 2 and 4 may be cloned by the following procedure.

Since a PHA synthase is an enzyme protein translated from a chromogene in Pseudomonas cichorii YN2 strain, a chromosome DNA containing a desired PHA synthase is first obtained. A chromosome DNA may be separated from YN2 strain cells by a known separation method. For example, YN2 strain is cultured in a LB medium or an M9 medium supplemented with an appropriate carbon source, disrupt and treated as described by, for example, Marmer et al. in Journal of Molecular Biology, Vol. 3, p. 208 (1961) to prepare a chromosome DNA.

Then, a gene library is prepared from the chromosome DNA thus obtained. The chromosome DNA is degraded using an appropriate restriction enzyme (e.g., Sau3AI) and a fragment with a proper length is ligated with a ligatable vector truncated with a restriction enzyme (e.g., BamHI) to prepare a gene library.

Depending on a vector used in preparing a library, a proper fragment length varies, e.g., about 4000 to 25000 bps for a usual plasmid vector and about 15000 to 30000 bps for a cosmid or phage vector. A proper length of DNA fragment may be collected by a known method such as a method using a sucrose density gradient or using an agarose gel described in Molecular Cloning, Cold Spring Harbor Laboratory (1982).

Since E. coli is used as a host microorganism in a gene library, a vector is a phage vector or plasmid vector which can autonomously grow in the host microorganism (E. coli). Examples of phage or cosmic vectors generally used include pWE15, M13, λEMBL3, λEMBL4, λFIXII, λDASHII, λZAPII, λgt10, λgt11, Charon4A and Charon21A. Examples of frequently used plasmid vectors include pBR, pUC, pBluescriptII, pGEM, pTZ and pET groups. In addition to E. coli, various shuttle vectors may be used, e.g., vectors which may autonomously replicate in a plurality of host microorganisms such as Pseudomonas sp. Again, these vectors may be, depending on a chromosome DNA to be ligated to them, truncated with a proper restriction enzyme to provide a desired fragment.

A chromosome DNA fragment may be ligated with a vector fragment using a DNA ligase. For example, a commercially available ligation kit (Takara Shuzo Co., Ltd., etc.) may be used. Thus, for example, various chromosome DNA fragments may be ligated with a plasmid vector fragment to prepare a mixture of recombinant plasmids comprising various DNA fragments (hereinafter, referred to as a "gene library").

In addition to a method using a proper length of chromosome DNA fragment, a gene library may be prepared by a method that all mRNAs are extracted from YN2 strain, purified and used for preparation of a cDNA fragment using a reverse transcriptase as described in Molecular Cloning, Cold Spring Harbor Laboratory, 1982. Alternatively, a prepared vector is used in a gene library to transform or transduce to E. coli, and then the host E. coli is cultured to amplify the gene library to a large amount as described in Molecular Cloning, Cold Spring Harbor Laboratory, 1982.

A recombinant vector comprising a gene DNA fragment may be introduced into a host microorganism by a known method. For example, when using E. coli as a host microorganism, a recombinant plasmid vector may be introduced using a calcium chloride method (Journal of Molecular Biology, Vol. 53, p. 159 (1970)), a rubidium chloride method (Methods in Enzymology, Vol. 68, p. 253 (1979)), electroporation (Current Protocols in Molecular Biology, Vol. 1, p. 1.8.4 (1994)). When using a cosmid vector or phage vector, transduction in a host E. coli may be conducted using in vitro packaging (Current Protocols in Molecular Biology, Vol. 1, p. 5.7.1 (1994)). Alternatively, conjugational transfer with a strain retaining a recombinant vector may be utilized to prepare a strain retaining a vector.

Then, from the gene library, a probe is prepared for obtaining a DNA fragment comprising a PHA synthase gene of YN2 strain.

Some base sequences have been reported for PHA synthase genes in known microorganisms; for example, Peoples, O. P. and Sinskey, A. J., J. Biol. Chem., 264, 15293 (1989); Huisman, G. W. et al., J. Biol. Chem., 266, 2191 (1991); Pieper, U. et al., FEMS Microbiol. Lett., 96, 73 (1992); Timm, A. and Steinbuchel, A., Eur. J. Biochem., 209, 15(1992); Matsusaki, H. et al., J. Bacteriol., 180, 6459 (1998). These reported sequences are compared to select a region where a sequence is preserved to a higher degree and thus to design an oligonucleotide for a primer used in polymerase chain reaction (hereinafter, referred to as "PCR"). Such oligonucleotides for a primer utilizing a common feature of PHA synthase genes include, but not limited to, a sequence described in Timm, A. and Steinbuchel, A., Eur. J. Biochem., 209, 15 (1992). An oligonucleotide may be synthesized using, for example, a commercially available DNA synthesizer such as Custom Synthesis Service, Amersham-Pharmacia Biotech, depending on a designed sequence.

For a PHA synthase gene derived from YN2 of this invention, synthetic DNAs having the sequences of SEQ ID NOs. 5 and 6 were designed.

Then, the designed oligonucleotide as a primer is subject to polymerase chain reaction (PCR) using a chromosome DNA in YN2 strain as a template to obtain a PCR amplified fragment. The PCR amplified fragment, which is derived from the primer, comprises a sequence common in PHA synthase genes at both ends. A partial sequence derived from the PHA synthase gene itself in YN2 strain as a template is contained between sequences complementary to the primer at both ends.

The PCR amplified fragment obtained is, therefore, almost 100 % homologous to the PHA synthase gene in YN2 strain and is expected to exhibit a higher S/N ratio as a probe in colony hybridization. In addition, it may facilitate stringency control of hybridization.

The above PCR amplified fragment is labeled with an appropriate reagent and used as a probe to colony-hybridize the above chromosome DNA library for selecting a recombinant E. coli strain retaining the PHA synthase gene (Current Protocols in Molecular Biology, Vol. 1, p. 6.0.3 (1994)). For example, the PCR amplified fragment may be labeled using a common detection system using a labeled enzyme or a commercially available kit such as AlkPhosDirect (Amersham-Pharmacia Biotech).

A recombinant E. coli strain retaining a gene fragment comprising a PHA synthase gene may be selected by, in addition to the above method using a gene type, a method using a phenotype where PHA synthesis is directly evaluated. Specifically, in expression of a PHA synthase from a retained PHA synthase gene in a recombinant E. coli strain, PHA is produced by the PHA synthase. PHA synthesis may be detected to select a recombinant E. coli strain in which the PHA synthase is expressed. PHA synthesis may be detected by, for example, staining with Sudan Black B (Archives of Biotechnology, Vol. 71, p. 283 (1970)) or determination of PHA accumulation by phase contrast microscopy.

A plasmid is collected from a recombinant E. coli selected by any of the above methods using an alkali method (Current Protocols in Molecular Biology, Vol. 1, p. 1.6.1 (1994)). The collected plasmid may be used to provide a DNA fragment comprising a PHA synthase gene or multiple DNA fragments partially containing a PHA synthase gene. The DNA fragment obtained may be sequenced by, for example, the Sanger's sequencing method (Molecular Cloning, Vol. 2, p. 13.3 (1989). Specifically, it may be conducted by a dye-primer method or a dye-terminator method using an automatic sequencer such as DNA Sequencer 377A (Parkin Elmer). Since the sequence of the vector itself in which the DNA fragment has been incorporated is known, the sequence of the DNA fragment cloned therein may be unequivocally analyzed.

After sequencing all the obtained DNA fragments comprising a PHA synthase gene, hybridization may be conducted using a DNA fragment prepared by an appropriate method such as chemical synthesis, PCR using a chromosome DNA as a template or degradation of a DNA fragment comprising the sequence with a restriction enzyme as a probe to provide a PHA synthase gene DNA of this invention.

The inventors have selected a gene translated into a PHA synthase exhibiting the above substrate specificity from YN2 strain according to the above procedure to find a PHA synthase comprising at least two amino acid sequences. Specifically, the inventors have found a PHA synthase gene collected from the chromosome DNA of YN2 strain and comprising the sequence of SEQ ID NO. 2 and a PHA synthase encoded by the gene and comprising the amino acid of SEQ ID NO. 1 as well as a PHA synthase gene comprising the sequence of SEQ ID NO. 4 and a PHA synthase encoded by the gene and comprising the amino acid of SEQ ID NO. 3.

A PHA synthase gene of the present invention may include a degenerated isomer encoding the same polypeptide which has the same amino acid sequence and is different in a degeneration codon. More specifically, it also includes a degenerated isomer by selection and conversion of a more frequently used degenerated codon encoding the same amino acid depending on a host. Besides the PHA synthase comprising the amino acid sequence of SEQ ID NO. 1 inherent in YN2 strain and the PHA synthase comprising the amino acid sequence of SEQ ID NO. 3, a PHA synthase of this invention may have mutation such as deletion, substitution and addition for several amino acids as long as its PHA producing activity and substrate specificity may not be deteriorated or the amino acid sequence may be maintained. Mutation such as deletion, substitution and addition may be introduced by a site mutation introduction technique based on a PHA synthase gene inherent in YN2 strain having the sequence of SEQ ID NO. 2 or 4 (Current Protocols in Molecular Biology Vol. 1, p. 8.1.1 (1994)).

A recombinant vector of the present invention is used in an application where a recombinant PHA synthase of this invention is expressed using Pseudomonas sp. or a microorganism such as E. coli as a host. It is, therefore, preferable that the recombinant vector of this invention itself can autonomously replicate in a host used while comprising a promoter for expression, a PHA synthase gene DNA of this invention and a transcription termination sequence suitable to the host. In addition, it is preferable that after introducing the recombinant vector, a vector comprising various marker genes used for its selection is used.

Expression vectors suitable to various types of bacterial hosts such as Pseudomonas sp. and E. coli include pLA2917 (ATCC 37355) having a RK2 replication origin which may be replicated and retained by a range of hosts or pJRD215 (ATCC 37533) having a RSF1010 replication origin. Without being limited to these, any vector having a replication origin which may be replicated and retained by a range of hosts may be used. Any promoter which may be expressed in a bacterium as a host may be used; for example, promoters derived from E. coli, a phage, etc. such as trp, trc, tac, lac, PL, PR, T7 and T3 promoters.

When using a yeast as a host, an expression vector may be YEp13, YCp50, pRS or pYEX vector. A promoter may be, for example, GAL or AOD promoter.

A transformed microorganism of this invention may be produced by introducing a recombinant vector of this invention into a host suitable to an expression vector used during preparing the recombinant vector. Examples of bacteria which may be used as a host include Esherichia sp., Pseudomonas sp., Ralstonia sp., Alcaligenes sp., Comamonas sp., Burkholderia sp., Agrobacterium sp., Flabobacterium sp., Vibrio sp., Enterobacter sp., Rhizobium sp., Gluconobacter sp., Acinetobacter sp., Moraxella sp., Nitrosomonas sp., Aeromonas sp., Paracoccus sp., Bacillus sp., Clostridium sp., Lactobacillus sp., Corynebacterium sp., Arthrobacter sp., Achromobacter sp., Micrococcus sp., Mycobacterium sp., Streptococcus sp., Streptomyces sp., Actinomyces sp., Norcadia sp. and Methylobacterium sp. A recombinant DNA may be introduced into a bacterium by an appropriate technique such as the above calcium chloride method and electroporation.

Besides the above bacteria, yeasts and molds such as Saccharomyces sp. and Candida sp. may be used as a host. A recombinant DNA may be introduced into an yeast by, for example, electroporation (Methods Enzymol., 194, 182-187 (1990)), a spheroplast method (Proc. Natl. Acad. Sci. USA, 84, 1929-1933 (1978)) and a lithium acetate method (J. Bacteriol., 153, 163-168 (1983)).

A PHA synthase of this invention may be prepared by culturing a transformant of this invention prepared by the above procedure and making a corresponding PHA synthase gene in an introduced expression vector producing the synthase as a recombinant protein. The PHA synthase of this invention is produced and accumulated in the culture (cultured bacterium or culture supernatant) and separated from the culture to be used for production of a recombinant enzyme protein. For this purpose, a transformant of this invention may be cultured by a usual procedure used for culturing a host. Culturing may be conducted by any of common methods used for culturing a microorganism such as batch, flow batch, continuous culturing and reactor styles. This culturing may be conducted by using, for example, a medium containing an inducer for expressing the above polyhydroxyalkanoate synthase gene.

For a transformant obtained using a bacterium such as E. coli as a host, a medium used for culturing may be a complete medium or synthetic medium such as LB medium and M9 medium. A microorganism may be grown by aerobically culturing at a culturing temperature of 25 to 37°C for 8 to 72 hours. Then, the bacteria are collected for obtaining a PHA synthase accumulated in them. Examples of a carbon source for the microorganism include sugars such as glucose, fructose, sucrose, maltose, galactose and starches; lower alcohols such as ethanol, propanol and butanol; polyalcohols such as glycerol; organic acids such as acetic acid, citric acid, succinic acid, tartaric acid, lactic acid and gluconic acid; and aliphatic acids such as propionic acid, butanoic acid, pentanoic acid, hexanoic acid, heptanoic acid, octanoic acid, nonanoic acid, decanoic acid, undecanoic acid and dodecanoic acid.

Examples of a nitrogen source include ammonia; ammonium salts such as ammonium chloride, ammonium sulfate and ammonium phosphate; and natural product derivatives such as peptone, meat extract, yeast extract, malt extract, casein decomposition products and corn steep liquor. Examples of an inorganic material include potassium dihydrogen phosphate, potassium monohydrogen phosphate, magnesium phosphate, magnesium sulfate and sodium chloride. The culture medium may contain an antibiotic such as kanamycin, ampicillin, tetracycline, chloramphenicol and streptomycin, depending on, for example, the type of a drug resistance gene used as a marker gene.

When using an inducible promoter in an expression vector, expression may be enhanced by adding a proper inducer depending on the type of the promoter during culturing a transformed microorganism. For example, the inducer may be isopropyl-β-D-thiogalactopyranoside (IPTG), tetracycline or indoleacrylic acid (IAA).

A PHA synthase may be separated and purified by centrifuging and collecting a culture obtained and processing it by a technique such as affinity chromatography, cation or anion exchange chromatography and gel filtration alone or in combination as appropriate. Whether a purified material is a desired enzyme is determined by a usual method such as SDS polyacrylamide gel electrophoresis and Western blotting.

This invention is not limited to the procedures as described above for culturing of a transformed microorganism of the present invention, production of a PHA synthase by the transformed microorganism of this invention and accumulating it in bacterial cells, and collection and purification of the PHA synthase from the cells.

A transformed microorganism of the present invention may be used for expressing a recombinant PHA synthase to produce a desired PHA by culturing thereof. For example, the microorganism may be cultured under the above culturing conditions to produce a recombinant PHA synthase while a substrate corresponding to the desired PHA on which the PHA synthase acts is added to a medium. Most conveniently, the PHA may be collected from the culture and the producing bacteria by extraction with an organic solvent commonly used such as chloroform. In an environment where using an organic solvent such as chloroform is undesirable, the culture may be treated a surfactant such as SDS, an enzyme such as lysozyme, or an agent such as EDTA, sodium hypochlorite and ammonia to remove bacterium components other than the PHA for collecting the PHA. This invention is not limited to the above procedures for culturing of a transformed microorganism of this invention for production of a PHA, production of a PHA by and accumulation thereof in a cultured microorganism, and collection of the PHA from a recombinant microorganism.

### Examples

This invention will be more specifically described with reference to Examples, although these Examples are illustrated as the best embodiments of this invention and do not limit the technical range of this invention.

### (Example 1) Cloning of a PHA synthase gene of YN2 strain

YN2 strain was cultured in 100 mL of LB medium (1 % polypeptone, 0.5 % yeast extract, 0.5 % sodium chloride, pH 7.4) at 30°C overnight and then a chromosome DNA was separated and collected as described by Marmer. The obtained chromosome DNA was completely digested using a restriction enzyme BglII. A vector pUC18 was cleaved with a restriction enzyme BamHI. After dephosphorylation of the terminals (Molecular Cloning, Vol. 1, p. 5.7.2 (1989), Cold Spring Harbor Laboratory), the cleaved site of the vector (cloning site) and the chromosome DNA fragment after BglII complete digestion were ligated using a DNA ligation kit Ver. II (Takara Shuzo Co., Ltd.). The plasmid vector in which the chromosome DNA fragment was integrated was used to transform Escheichia coli HB101 for preparing a chromosome DNA library for YN2 strain.

Then, in order to select a DNA fragment comprising a PHA synthase gene of YN2 strain, a probe for colony hybridization was prepared. An oligonucleotide consisting of the sequences of SEQ ID NOs. 5 and 6 (Amersham-Pharmacia Biotech) was prepared and used as a primer for PCR using the chromosome DNA as a template. A PCR-amplified DNA fragment was used as a probe. Labeling of the probe was conducted using a commercially available labeling enzyme system AlkPhosDirect (Amersham-Pharmacia Biotech). The labeled probe thus obtained was used to select an E. coli strain containing a recombinant plasmid comprising the PHA synthase gene from the chromosome DNA library of YN2 strain by colony hybridization. From the selected strain, the plasmid was collected by an alkali method to prepare a DNA fragment comprising a PHA synthase gene.

The gene DNA fragment thus obtained was recombined in a vector pBBR122 (Mo BiTec) comprising a wide host range of replication region which did not belong to IncP, IncQ or IncW in an incompatible group. The recombinant plasmid was transformed in Pseudomonas cichorii YN2m1 strain (a strain depleted of PHA synthesizing ability) by electroporation, and then the YN2m1 strain regained PHA synthesizing ability and exhibited complementarily. It demonstrates that the selected gene DNA fragment comprises a region of a PHA synthase gene translatable into a PHA synthase in Pseudomonas cichorii YN2m1.

The DNA fragment comprising a PHA synthase gene was sequenced by the Sanger's sequencing method. It was thus found that the determined sequence comprised the sequences of SEQ ID NOs. 2 and 4 each of which encoded a peptide chain. As described below, it was determined that both proteins consisting of a peptide chain had enzyme activity and that the sequences of SEQ ID NOs. 2 and 4 were therefore PHA synthase genes. Specifically, it was found that the sequences of SEQ ID NOs. 2 and 4 encoded the amino acid sequences of SEQ ID NOs. 1 and 3, respectively, and that a protein comprising one of these amino acid sequences alone could produce a PHA.

### (Example 2) Recombination of a PHA synthase gene of YN2 strain to an expression vector

A PHA synthase gene having the sequence of SEQ ID NO. 2 was PCRed using a chromosome DNA as a template to reproduce the whole length of a PHA synthase gene. An oligonucleotide having a sequence which was an upstream primer to the sequence of SEQ ID NO. 2 and had a sequence upstream of its initiation codon (SEQ ID NO. 7) and an oligonucleotide having a sequence which was a downstream primer to the sequence of SEQ ID NO. 2 and had a sequence downstream of its termination codon (SEQ ID NO. 8) were designed and prepared (Amersham-Pharmacia Biotech). Using these oligonucleotides as a primer, PCR was conducted to amplify the whole length of the PHA synthase gene (LA-PCR kit; Takara Shuzo Co., Ltd.).

Likewise, a PHA synthase gene having the sequence of SEQ ID NO. 4 was PCRed using a chromosome DNA as a template to reproduce the whole length of a PHA synthase gene. An oligonucleotide having a sequence which was an upstream primer to the sequence of SEQ ID NO. 4 and had a sequence upstream of its initiation codon (SEQ ID NO. 9) and an oligonucleotide having a sequence which was a downstream primer to the sequence of SEQ ID NO. 4 and had a sequence downstream of its termination codon (SEQ ID NO. 10) were designed and prepared (Amersham-Pharmacia Biotech). Using these oligonucleotides as a primer, PCR was conducted to amplify the whole length of the PHA synthase gene (LA-PCR kit; Takara Shuzo Co., Ltd.).

Each of the obtained PCR amplified fragment containing the whole length of the PHA synthase gene was completely digested using a restriction enzyme HindIII. Separately, an expression vector pTrc99A was also truncated with a restriction enzyme HindIII and dephosphorylated (Molecular Cloning, Vol. 1, p. 5.7.2 (1989), Cold Spring Harbor Laboratory). To the truncated site of the expression vector pTrc99A was ligated the DNA fragment comprising the whole length of the PHA synthase gene from which unnecessary sequences had been removed at both ends, using a DNA ligation kit Ver. II (Takara Shuzo Co., Ltd.).

Using the recombinant plasmids obtained, Escherichia coli HB101 (Takara Shuzo Co., Ltd.) was transformed by a calcium chloride method. The recombinants were cultured, and the recombinant plasmids were amplified and collected individually. The recombinant plasmid retaining the gene DNA of SEQ ID NO. 2 was designated pYN2-C1 (derived from SEQ ID NO. 2) while the recombinant plasmid retaining the gene DNA of SEQ ID NO. 4 was designated pYN2-C2 (derived from SEQ ID NO. 4).

### (Example 3) PHA production (1) using a PHA synthase gene recombinant E. coli

Using the recombinant plasmids obtained in Example 2, pYN2-C1 (derived from SEQ ID NO. 2) and pYN2-C2 (derived from SEQ ID NO. 4), an Escherichia coli HB101fB (fadB deficient strain) was transformed by a calcium chloride method to prepare recombinant E. coli strains retaining the recombinant plasmid, pYN2-C1 and pYN2-C2 recombinant strains, respectively.

Each of the pYN2-C1 and pYN2-C2 recombinant strains was inoculated to 200 mL of M9 medium containing 0.5 % yeast extract and 0.1 % FPVA, and the medium was shaken at 37°C with a rate of 125 strokes/min. After 24 hours, the cells were collected by centrifugation, washed once with cold methanol and lyophilized.

The lyophilized pellet was suspended in 100 mL of chloroform and the suspension was stirred at 60°C for 20 hours to extract a PHA. After filtering the extract through a membrane filter with a pore size of 0.45 µm, the filtrate was concentrated by rotary evaporation. Then, the concentrate was re-suspended in cold methanol and the precipitant was collected and dried in vacuo to provide a PHA. The PHA thus obtained was subject to methanolysis as usual and analyzed using a gas chromatography-mass spectrometry apparatus (GC-MS, Shimadzu QP-5050, EI technique) to identify methyl-esterified PHA monomer units. Table 1 shows together a cell dry weight, a polymer dry weight for a collected PHA, a polymer yield per a cell (polymer dry weight/cell dry weight) and identities of monomer units for each strain.

**Table 1**

| | pYN2-C1 recombinant strain | pYN2-C2 recombinant strain |
|---|---|---|
| Cell dry weight | 1150 mg/L | 1200 mg/L |
| Polymer dry weight | 101 mg/L | 110 mg/L |
| Polymer dry weight/Cell dry weight | 9% | 9 % |
| Monomer unit composition (area ratio) | | |
| 3-Hydroxybutyric acid | 0 % | 0 % |
| 3-Hydroxyvateric acid | 0% | 0 % |
| 3-Hydroxyhexanoic acid | 0 % | 0 % |
| 3-Hydroxyheptanoic acid | 3% | 3 % |
| 3-Hydroxyoctanoic acid | 8 % | 10 % |
| 3-Hydroxynonanoic acid | 2 % | 1 % |
| 3-Hydroxydecanoic acid | 12% | 10 % |
| 3-Hydroxy-5-(4-fluorophenyl) valeric acid | 75% | 76 % |

These results show that both pYN2-C1 and pYN2-C2 recombinant strains produce, from the substrate 5-(4-fluorophenyl) valeric acid, PHAs comprising a monomer unit represented by formula (I) derived from corresponding 3-hydroxy-5-(4-fluorophenyl) valeric acid as a main component. It is, therefore, demonstrated that although the pYN2-C1 and pYN2-C2 recombinant strains exclusively produce PHA synthases having the amino acid sequences of SEQ ID NOs. 1 and 3 translated from the PHA synthase genes comprising the sequences of SEQ ID NOs. 2 and 4, respectively, both strains similarly convert the substrate 5-(4-fluorophenyl) valeric acid into the monomer unit represented by formula (I) derived from corresponding 3-hydroxy-5-(4-fluorophenyl) valeric acid and produce a PHA containing the monomer unit.

### (Example 4) PHA production (2) using a PHA synthase gene recombinant E. coli

Using the recombinant plasmids obtained in Example 2, YN2-C1 (derived from SEQ ID NO. 2) AND YN2-C2 (derived from SEQ ID NO. 4), an Escherichia coli HB101fB (fadB deficient strain) was transformed by a calcium chloride method to prepare recombinant E. coli strains from the above recombinant plasmid.

Each of the pYN2-C1 and pYN2-C2 recombinant strains was inoculated to 200 mL of M9 medium containing 0.5 % yeast extract and 0.1 % 4-cyclohexylbutyric acid, and the medium was shaken at 37°C with a rate of 125 strokes/min. After 24 hours, the cells were collected by centrifugation, washed once with cold methanol and lyophilized.

The lyophilized pellet was suspended in 100 mL of chloroform and the suspension was stirred at 60°C for 20 hours to extract a PHA. After filtering the extract through a membrane filter with a pore size of 0.45 µm, the filtrate was concentrated by rotary evaporation. Then, the concentrate was re-suspended in cold methanol and the precipitant was collected and dried in vacuo to provide a PHA. The PHA thus obtained was subject to methanolysis as usual and analyzed using a gas chromatography-mass spectrometry apparatus (GC-MS, Shimadzu QP-5050, EI technique) to identify methyl-esterified PHA monomer units. Table 2 shows together a cell dry weight, a polymer dry weight for a collected PHA, a polymer yield per a cell (polymer dry weight/cell dry weight) and identities of monomer units for each strain.

**Table 2**

| | pYN2-C1 recombinant strain | pYN2-C2 recombinant strain |
|---|---|---|
| Cell dry weight | 900 mg/L | 880 mg/L |
| Polymer dry weight | 55 mg/L | 52 mg/L |
| Polymer dry weight/Cell dry weight | 6% | 6 % |
| Monomer unit composition (area ratio) | | |
| 3-Hydroxybutyric acid | 0% | 0% |
| 3-Hydroxyvaleric acid | 0% | 0% |
| 3-Hydroxyhexanoic acid | 0% | 0 % |
| 3-Hydroxyheptanoic acid | 1 % | 0% |
| 3-Hydroxyoctanoic acid | 4% | 5% |
| 3-Hydroxynonanoic acid | 2 % | 1 % |
| 3-Hydroxydecanoic acid | 9 % | 8 % |
| 3-Hydroxy-4-cydohexylbutyric acid | 84% | 86% |

These results show that both pYN2-C1 and pYN2-C2 recombinant strains produce, from the substrate 4-cyclohexylbutyric acid, PHAs comprising a monomer unit represented by formula (VI) derived from corresponding 3-hydroxy-4-cyclohexylbutyric acid as a main component. It is, therefore, demonstrated that although the pYN2-C1 and pYN2-C2 recombinant strains exclusively produce PHA synthases having the amino acid sequences of SEQ ID NOs. 1 and 3 translated from the PHA synthase genes comprising the sequences of SEQ ID NOs. 2 and 4, respectively, both strains similarly convert the substrate 4-cyclohexylbutyric acid into the monomer unit represented by formula (IX) derived from corresponding 3-hydroxy-4-cyclohexylbutyric acid and produce a PHA containing the monomer unit.

The results together with those in Example 3 demonstrate that the PHA synthases having the amino acid sequences of SEQ ID NOs. 1 and 3 have enzyme activity mutually similar in substrate specificity.

## Claims

1. A polyhydroxyalkanoate synthase having the amino acid sequence of SEQ ID NO. 1.

2. A polyhydroxyalkanoate synthase having an amino acid sequence wherein the amino acid sequence of SEQ ID NO. 1 has been modified by deleting, substituting or adding amino acids to such extent that the resulting polyhydroxyalkanoate synthase still has the activity to convert 5-(4-fluorphenyl)valeric acid into a polymer having a monomer composition wherein the main component is made of 3-hydroxy-5-(4-fluorphenyl)valeric acid.

3. A polyhydroxyalkanoate synthase having an amino acid sequence wherein the amino acid sequence of SEQ ID NO. 1 has been modified by deleting, substituting or adding amino acids to such extent that the resulting polyhydroxyalkanoate synthase still has the activity to convert 4-cyclohexylbutyric acid into a polymer having a monomer composition wherein the main component is made of 3-hydroxy-4-cyclohexylbutyric acid.

4. A polyhydroxyalkanoate synthase gene comprising a DNA sequence encoding the amino acid sequence of the polyhydroxyalkanoate synthase according to claims 2 or 3.

5. The polyhydroxyalkanoate synthase gene comprising a DNA sequence of SEQ ID NO. 2 encoding the amino acid sequence of SEQ ID NO. 1.

6. A polyhydroxyalkanoate synthase having the amino acid sequence of SEQ ID NO, 3.

7. A polyhydroxyalkanoate synthase having an amino acid sequence wherein the amino acid sequence of SEQ ID NO. 3 has been modified by deleting, substituting or adding amino acids to such extent that the resulting polyhydroxyalkanoate synthase still has the activity to convert 5-(4-fluorphenyl)valeric acid into a polymer having a monomer composition wherein the main component is made of 3-hydroxy-5-(4-fluorphenyl)valeric acid.

8. A polyhydroxyalkanoate synthase having an amino acid sequence wherein the amino acid sequence of SEQ ID NO. 3 has been modified by deleting, substituting or adding amino acids to such extent that the resulting polyhydroxyalkanoate synthase still has the activity to convert 4-cyclohexylbutyric acid into a polymer having a monomer composition wherein the main component is made of 3-hydroxy-4-cyclohexylbutyric acid.

9. A polyhydroxyalkanoate synthase gene comprising a DNA sequence encoding the amino acid sequence of the polyhydroxyalkanoate synthase according to claims 7 or 8.

10. The polyhydroxyalkanoate syntase gene comprising a DNA sequence of SEQ ID NO. 4 encoding the amino acid sequence of SEQ ID NO. 3.

11. A recombinant vector comprising the gene according to claim 4, 5, 9 or 10 as a polyhydroxyalkanoate synthase gene.

12. A transformed microorganism transformed by introduction of the recombinant vector according to claim 11.

13. A method for preparing a polyhydroxyalkanoate comprising the steps of culturing the transformed microorganism according to claim 1 2 in a medium containing a substrate for a polyhydroxyalkanoate synthase and isolating the polyhydroxyalkanoate from the culture obtained.

14. A method for producing a polyhydroxyalkanoate synthase comprising the steps of culturing the transformed microorganism according to claim 12 and causing the transformed microorganism to produce the polyhydroxyalkanoate synthase.

## Patentansprüche

1. Polyhydroxyalkanoat-Synthase, die die Aminosäuresequenz mit der Sequenzidentifizierungsnummer 1 aufweist.

2. Polyhydroxyalkanoat-Synthase, die eine Aminosäuresequenz aufweist, wobei die Aminosäuresequenz der Sequenzidentifizierungsnummer 1 durch Deletion, Substitution oder Hinzufügen von Aminosäuren soweit modifiziert worden ist, daß die entstandene Polyhydroxyalkanoat-Synthase noch die Aktivität aufweist, um 5-14-Fluorphenyl)valeriansäure in ein Polymer zu überführen, das eine Monomerzusammensetzung hat, bei der die Hauptkomponente aus 3-Hydroxy-5-(4-fluorphenyl)valeriansäure erzeugt worden ist.

3. Polyhydroxyalkanoat-Synthase, die eine Aminosäuresequenz aufweist, wobei die Aminosäuresequenz der Sequenzidentifizierungsnummer 1 durch Deletion, Substitution oder Hinzufügen von Aminosäuren soweit modifiziert worden ist, daß die entstandene Polyhydroxyalkanoat-Synthase noch die Aktivität aufweist, um 4-Cyclohexylbuttersäure in ein Polymer zu überführen, das eine Monomerzusammensetzung hat, bei der die Hauptkomponente aus 3-Hydroxy-4-cyclohexylbuttersäure erzeugt worden ist.

4. Polyhydroxyalkanoat-Synthase-Gen, umfassend eine DNA-Sequenz, die die Aminosäuresequenz der Polyhydroxyalkanoat-Synthase nach Anspruch 2 oder 3 codiert.

5. Polyhydroxyalkanoat-Synthase-Gen, umfassend eine DNA-Sequenz der Sequenzidentifizierungsnummer 2, die die Aminosäuresequenz der Sequenzidentifizierungsnummer 1 codiert.

6. Polyhydroxyalkanoat-Synthase, die die Aminosäuresequenz der Sequenzidentifizierungsnummer 3 aufweist.

7. Polyhydroxyalkanoat-Synthase, die eine Aminosäuresequenz aufweist, wobei die Aminosäuresequenz der Sequenzidentifizierungsnummer 3 durch Deletion, Substitution oder Hinzufügen von Aminosäuren soweit modifiziert worden ist, daß die entstandene Polyhydroxyalkanoat-Synthase noch die Aktivität aufweist, um 5-(4-Fluorphenyl)valeriansäure in ein Polymer zu überführen, das eine Monomerzusammensetzung hat, bei der die Hauptkomponente aus 3-Hydroxy-5-(4-fluorphenyl)valeriansäure erzeugt worden ist.

8. Polyhydroxyalkanoat-Synthase, die eine Aminosäuresequenz aufweist, wobei die Aminosäuresequenz der Sequenzidentifizierungsnummer 3 durch Deletion, Substitution oder Hinzufügen von Aminosäuren soweit modifiziert worden ist, daß die entstandene Polyhydroxyalkanoat-Synthase noch die Aktivität aufweist, um 4-Cyclohexylbuttersäure in ein Polymer zu überführen, das eine Monomerzusammensetzung hat, bei der die Hauptkomponente aus 3-Hydroxy-4-cyclohexylbuttersäure erzeugt worden ist.

9. Polyhydroxyalkanoat-Synthase-Gen, umfassend eine DNA-Sequenz, die die Aminosäuresequenz der Polyhydroxyalkanoat-Synthase nach Anspruch 7 oder 8 codiert.

10. Polyhydroxyalkanoat-Synthase-Gen, umfassend eine DNA-Sequenz der Sequenzidentifizierungsnummer 4, die die Aminosäuresequenz der Sequenzidentifizierungsnummer 3 codiert.

11. Rekombinanter Vektor, umfassend das Gen nach Anspruch 4, 5, 9 oder 10 als ein Polyhydroxyalkanoat-Synthase-Gen.

12. Transformierter Mikroorganismus, der durch Einführen des rekombinanten Vektors nach Anspruch 11 transformiert worden ist.

13. Verfahren zur Herstellung eines Polyhydroxyalkanoats, umfassend die Schritte:
Züchten des transformierten Mikroorganismus nach Anspruch 12 in einem Medium, das ein Substrat für eine Polyhydroxyalkanoat-Synthase enthält, und Abtrennen des Polyhydroxyalkanoats von der erhaltenen Kultur.

14. Verfahren zur Herstellung von Polyhydroxyalkanoat-Synthase, umfassend die Schritte:
Züchten des transformierten Mikroorganismus nach Anspruch 12 und
Bewirken, daß der transformierte Mikroorganismus die Polyhydroxyalkanoat-Synthase produziert.

## Revendications

1. Polyhydroxyalcanoate-synthase ayant la séquence d'aminoacides de la SEQ ID N°1.

2. Polyhydroxyalcanoate-synthase ayant une séquence d'aminoacides dans laquelle la séquence d'aminoacides de la SEQ ID N° 1 a été modifiée par délétion, substitution ou addition d'aminoacides de telle sorte que la polyhydroxyalcanoate-synthase résultante possède encore l'activité de conversion de l'acide 5-(4-fluorophényl)valérique en un polymère ayant une composition en monomères dans laquelle le constituant principal est formé d'acide 3-hydroxy-5-(4-fluorophényl)valérique.

3. Polyhydroxyalcanoate-synthase ayant une séquence d'aminoacides dans laquelle la séquence d'aminoacides de la SEQ ID N° 1 a été modifiée par délétion, substitution ou addition d'aminoacides de telle sorte que la polyhydroxyalcanoate-synthase résultante possède encore l'activité de conversion de l'acide 4-cyclohexylbutyrique en un polymère ayant une composition en monomères dans laquelle le constituant principal est formé d'acide 3-hydroxy-4-cyclohexylbutyrique.

4. Gène de polyhydroxyalcanoate-synthase comprenant une séquence d'ADN codant pour la séquence d'aminoacides de la polyhydroxyalcanoate-synthase suivant les revendications 2 ou 3.

5. Gène de polyhydroxyalcanoate-synthase comprenant une séquence d'ADN de la SEQ ID N° 2 codant pour la séquence d'aminoacides de la SEQ ID N° 1.

6. Gène de polyhydroxyalcanoate-synthase ayant la séquence d'aminoacides de la SEQ ID N° 3.

7. Gène de polyhydroxyalcanoate-synthase ayant une séquence d'aminoacides dans laquelle la séquence d'aminoacides de la SEQ ID N° 3 a été modifiée par délétion, substitution ou addition d'aminoacides de telle sorte que la polyhydroxyalcanoate-synthase résultante possède encore l'activité de conversion de l'acide 5-(4-fluoro-phényl)valérique en un polymère ayant une composition en monomères dans laquelle le constituant principal est formé d'acide 3-hydroxy-5-(4-fluorophényl)-valérique.

8. Polyhydroxyalcanoate-synthase ayant une séquence d'aminoacides dans laquelle la séquence d'aminoacides de la SEQ ID N° 3 a été modifiée par délétion, substitution ou addition d'aminoacides de telle sorte que la polyhydroxyalcanoate-synthase résultante possède encore l'activité de conversion de l'acide 4-cyclohexylbutyrique en un polymère ayant une composition en monomères dans laquelle le constituant principal est formé d'acide 3-hydroxy-4-cyclohexylbutyrique.

9. Gène de polyhydroxyalcanoate-synthase comprenant une séquence d'ADN codant pour la séquence d'aminoacides de la polyhydroxyalcanoate-synthase suivant les revendications 7 ou 8.

10. Gène de polyhydroxyalcanoate-synthase comprenant une séquence d'ADN de la SEQ ID N° 4 codant pour la séquence d'aminoacides de la SEQ ID N° 3.

11. Vecteur recombinant comprenant le gène suivant la revendication 4, 5, 9 ou 10 comme gène de polyhydroxyalcanoate-synthase.

12. Micro-organisme transformé, qui a été transformé par l'introduction du vecteur recombinant suivant la revendication 11.

13. Procédé pour la préparation d'un polyhydroxyalcanoate, comprenant les étapes de culture du micro-organisme transformé suivant la revendication 12 dans un milieu contenant un substrat pour une polyhydroxyalcanoate-synthase et d'isolement du polyhydroxyalcanoate de la culture obtenue.

14. Procédé pour la production d'une polyhydroxyalcanoate-synthase, comprenant les étapes consistant à cultiver le micro-organisme transformé suivant la revendication 12 et à provoquer la production par le micro-organisme transformé de la polyhydroxyalcanoate-synthase.
